# FASCICULE DE BREVET EUROPEEN

(11) **EP 0 772 981 B1**
(45) Date de publication et mention de la délivrance du brevet: **18.07.2001**
(21) Numéro de dépôt: 96402319.6
(22) Date de dépôt: 31.10.1996
(51) Int. Cl.: A23L 3/015, A61L 2/02

(54) **Presse isostatique pour traitement en vrac à haute pression notamment de produits alimentaires liquides chargés de particules**
Isostatische Presse für Hochdruckbehandlung von Schüttgut, insbesondere von mit Partikeln beladenen flüssigen Lebensmitteln
Isostatic press for high-pressure treatment of bulk material, especially of liquid food products charged with particles

(30) Priorité: 09.11.1995 FR 9513276
(43) Date de publication de la demande: 14.05.1997
(73) Titulaire: GEC ALSTHOM ACB, 75116 PARIS (FR)
(72) Inventeur: Ollivaud, Bernard, 44850 Le Cellier (FR)
(74) Mandataire: Gosse, Michel

(56) Documents cités:
- WO-A-94/28745
- FR-A- 2 442 018
- FR-A- 2 687 590
- FR-A- 2 690 854
- US-A- 5 288 462

## Description

La présente invention est relative à une presse isostatique pour le traitement à haute pression de produits liquides alimentaires, pharmaceutiques ou cosmétiques. L'invention concerne plus particulièrement le traitement en vrac de produits liquides chargés de particules.

On sait qu'en soumettant un produit tel qu'un produit alimentaire à une pression comprise par exemple entre 2000 et 10000 bars pendant une durée de quelques minutes, il en résulte une stérilisation du produit, qui permet ainsi d'accroître de manière importante sa durée de conservation.

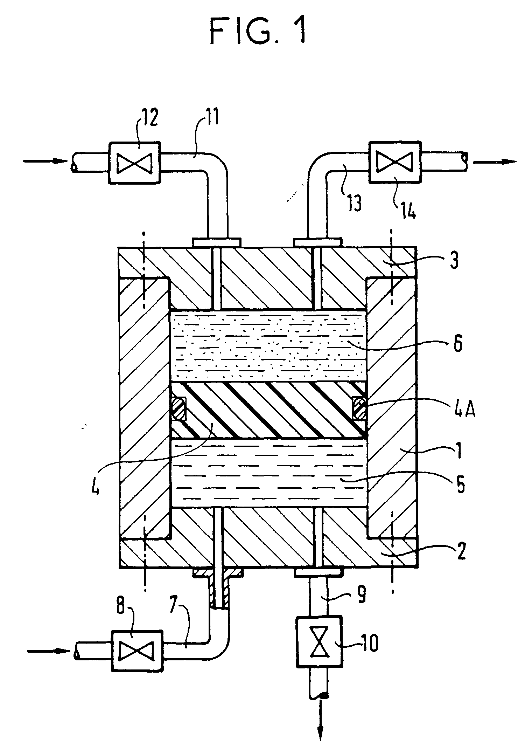

La figure 1 est un schéma d'une presse utilisée pour la mise en oeuvre du procédé de stérilisation ci-dessus. Elle comprend essentiellement un cylindre 1, fermé hermétiquement par un fond inférieur 2 et un fond supérieur 3, et contenant en son intérieur un piston de révolution 4, muni d'un joint 4A, partageant l'intérieur du cylindre en une première chambre 5 et une seconde chambre 6.

La première chambre 5 est alimentée en fluide de pressurisation, par exemple de l'eau, par une conduite 7 équipée d'une vanne 8. Une conduite 9 équipée d'une vanne 10 permet la vidange de la chambre.

La seconde chambre est alimentée en produit à traiter, par exemple du jus de fruit, par une conduite 11 munie d'une vanne 12. Une conduite 13, équipée d'une vanne 14, permet l'évacuation du produit après son traitement à haute pression.

Ce traitement consiste à soumettre le produit pendant quelques minutes (1 à 10 par exemple) à une pression comprise entre 2000 et 10.000 bars.

Un inconvénient de la presse représentée figure 1 provient du fait que si l'on veut avoir des temps de cycles courts, il faut que les opérations de remplissage et de vidange de la chambre de traitement 6 soient brèves ; comme ce remplissage et cette vidange s'effectuent à faible pression, si l'on veut un grand débit il faut alors que la section de passage des vannes 12 et 14, et donc également des canalisations 11 et 13, soit grande. Cependant, lors de la mise sous très haute pression de la chambre 6 pour le traitement du produit, les vannes 12 et 14 ainsi que les conduites 11 et 13 sont soumises à cette pression, ce qui exige un dimensionnement en conséquence. Il en est de même de la vanne 10 et de la conduite 9 soumises à cette forte pression mais qui, lors de la vidange de la chambre 5 n'est soumise qu'à la faible pression de vidange.

Il est donc nécessaire de surdimensionner fortement les sections des vannes 9, 12 et 14 et des conduites 9, 11, 13 uniquement pour les opérations de remplissage et/ou de vidange mais aussi la section des parois pour résister à l'effort de la très haute pression, ou bien alors il faut se contenter de sections beaucoup plus faibles, mais au détriment d'un allongement de cycle lors des remplissages et des vidanges. Pour la conduite 7 et la vanne 8, le problème ne se pose pas puisqu'il s'agit de l'alimentation à très haute pression et donc la section peut être faible tout en conservant un bon débit.

WO-A-9 428 745 enseigne une presse pour le traitement à haute pression comportant au moins un cylindre et un piston, dont les moyens de connection sont situés hors de la chambre à haute pression.

La présente invention a pour but de pallier cet inconvénient et a ainsi pour objet une presse isostatique, notamment pour le traitement sous haute pression de produits alimentaires pharmaceutiques ou cosmétiques, comprenant un cylindre (1) fermé hermétiquement par un fond inférieur (2) et un fond supérieur (3) et contenant en son intérieur un piston libre (4) de révolution partageant l'intérieur du cylindre en une première chambre (5) et en une seconde chambre (6), ladite première chambre (5) étant munie d'une conduite (7) d'alimentation en fluide de pressurisation équipée d'une première vanne (8) et de moyens de vidange équipés d'une seconde vanne (10) et la seconde chambre (6) étant munie de moyens d'alimentation et de vidange pour le produit à traiter équipés respectivement d'une troisième vanne et d'une quatrième vanne, caractérisée en ce que lesdites seconde, troisième et quatrième vannes comportent chacune un siège de vanne (27, 27A, 27B) situé à l'extrémité interne d'un alésage (21, 21A, 21B) d'alimentation ou de vidange de la chambre correspondante (5, 6) traversant le fond correspondant supérieur (3) ou inférieur (2), ledit siège coopérant avec un clapet (24-25, 24A-25A, 24B, 25B) disposé intérieurement audit cylindre (1).

Dans un mode particulier de réalisation, l'alésage est pratiqué dans une pièce amovible vissée dans un passage fileté du fond.

L'alésage est pratiqué à travers le fond qui est monté flottant sur le cylindre et coopère avec une maille pour le report des efforts exercés par le fluide sous pression dans le cylindre.

L'étanchéité entre la pièce amovible ou le fond et le cylindre est assurée par un systéme de joints disposé dans un plan sensiblement confondu avec le plan où s'opère l'étanchéité entre le siège et le clapet.

Un exemple de mise en oeuvre de l'invention est décrit maintenant en référence au dessin annexé dans lequel:
- la figure 1 est un schéma d'une presse pour le traitement de produits alimentaires, selon l'art antérieur,
- la figure 2 est une vue partielle en coupe d'une presse selon un premier mode de réalisation de l'invention,
- la figure 3 est une vue en coupe d'une presse selon une variante de réalisation de l'invention,
- la figure 4 est une vue agrandie de la partie B de la Fig.3.

La Fig. 1 a déjà été commentée et on n'y reviendra pas.

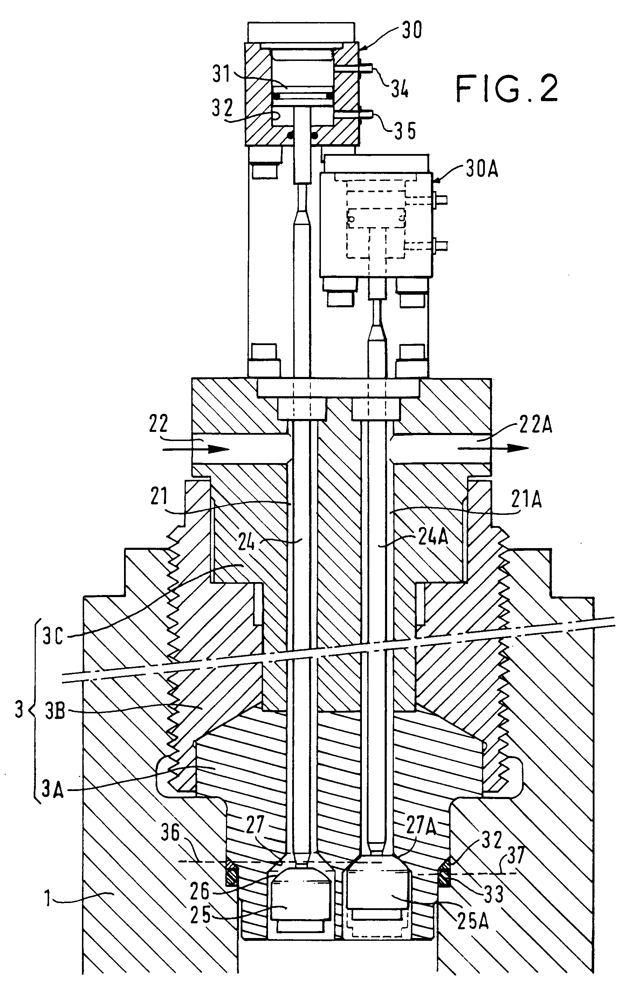

Les dispositions de l'invention sont décrites et expliquées en référence à la Fig.2.

La portion de presse représentée dans la Fig.2 correspond au fond supérieur 3 et aux vannes 12 et 14.

Le fond 3 est constitué de trois pièces assemblées 3A, 3B et 3C percé de deux alésages 21 et 21A communiquant avec des passages transversaux 22 d'alimentation en produit à traiter et 22A d'évacuation du produit traité.

A l'intérieur de l'alésage 21 est disposée axialement une tige 24; la tige 24 porte à une première extrémité (côté chambre 6) un clapet 25, ayant une surface 26 pouvant venir en contact avec un siège constitué par une portion conique terminale 27 de l'alésage 21. La surface 26 est de préférence ovoïde.

La surface 27 et l'ensemble tige-clapet 24-25 constituent une vanne fermant la chambre 6 sur le circuit de chargement. Cette vanne sera dénommée dans la suite vanne de chargement et désignée par la référence 24-25. Elle correspond à la vanne 12 de la figure 1.

L'alésage 21A comporte les mêmes éléments que l'alésage 21, désignés cette fois avec le suffixe A, constituant une vanne sur le circuit d'évacuation. Cette vanne sera dénommée dans la suite vanne d'évacuation et désignée par la référence 24A-25A et elle correspond à la vanne 14 de la figure 1.

La manoeuvre des vannes s'effectue grâce à des vérins 30 et 30A.

Un exemple de réalisation du vérin 30 est décrit maintenant. Le vérin 30 comprend un piston 31 se déplaçant à l'intérieur d'un cylindre 32. Le piston 31 est fixé à la seconde extrémité de la tige 24. Le vérin, à double effet, est actionné par un fluide de travail pénétrant de part et d'autre du piston 31 par des conduits 34 et 35 respectivement.

La pièce 3A qui comporte les deux sièges 27 et 27A est démontable pour permettre son changement lors des opérations de maintenance.

Le fonctionnement de la presse est le suivant:
- les vannes de remplissage 24-25 et d'évacuation 24A-25A sont successivement ouvertes par action des vérins 30 et 30A.
- le remplissage de la chambre 6 en produit à traiter est effectué par le passage d'alimentation 22, et le piston 4 est chassé vers le bas.
- une fois le remplissage terminé, les vannes 24-25 et 24A-25A sont fermées.
- la haute pression est mise sur le produit par action du piston 4 qui est poussé vers sa partie supérieure par un fluide de compression injecté à travers la vanne 8 (figure 1). Les clapets 25 et 25A sont fortement appuyés sur leur siège 27 et 27A grâce à la pression règnant dans la chambre 6, la pression dans les alésages 21 et 21A étant la pression atmosphérique.
- après traitement, la haute pression est supprimée dans la chambre 6 par fermeture de la vanne 8 et ouverture de la vanne 10, et la vanne d'évacuation 24A-25A est ouverte par manoeuvre du vérin 30A. On voit que les vérins 30 peuvent être de faible puissance puisqu'ils n'ont à vaincre aucune haute pression, mais qu'ils ont simplement pour rôle de déplacer respectivement les ensembles tige-clapet 24-25 et 24A-25A.

Bien entendu, conformément à l'invention, la vanne 10 de vidange du fluide de mise en pression est également située à l'intérieur du fond inférieur 2 comme les clapets 24-25 et 24A-25A. Une figure n'a pas été spécialement faite pour cela.
- le liquide traité de la chambre est ensuite évacué, par l'action du piston 4, à travers le conduit 22A en fermant la vanne 10 et en ouvrant la vanne 8. La grande section de passage d'une part entre le siège 27 et le clapet 25 et d'autre part entre le siège 27A et le clapet 25A, qui, par exemple, ne sont pas inférieures à 200 mm², permettent un chargement et une évacuation rapides du liquide en traitement, ce qui entraîne une diminution notable de la durée du cycle de traitement du produit. Au passage entre le siège 27 et le clapet 25, le liquide traité tourbillonne, ce qui a pour effet d'entraîner les particules solides qui auraient pu se coller sur le siège ou sur le clapet. Cet auto-nettoyage garantit une bonne étanchéité de la vanne, cycle après cycle.

On rappelle que les sièges 27 et 27A sont réalisés dans une partie 3A du fond 3, qui est amovible. Cette partie 3A peut être changée lorsque l'on constate que le contact entre le clapet et son siège a tendance à se détériorer.

Par ailleurs, la pièce 3A porte un système de joints 32 et 33 assurant l'étanchéité entre la chambre 6 et l'extérieur. Ces pièces peuvent également être remplacées lors de la dépose de la pièce 3A.

L'étanchéité entre le clapet 25 et son siège 27 (ou entre le clapet 25A et son siège 27A) se fait dans un plan horizontal représenté par la ligne 36 en traits tiretés. L'étanchéité entre le fond 3 (au niveau de la pièce 3A) et le corps cylindrique 1 de la presse se fait selon un plan 37 qui correspond à l'élément de joint 32. Selon une caractéristique de l'invention, les plans 36 et 37 se situent à un même niveau. Le fait de placer les étanchéités sur un même plan permet de minimiser les contraintes des pièces soumises à la pression.

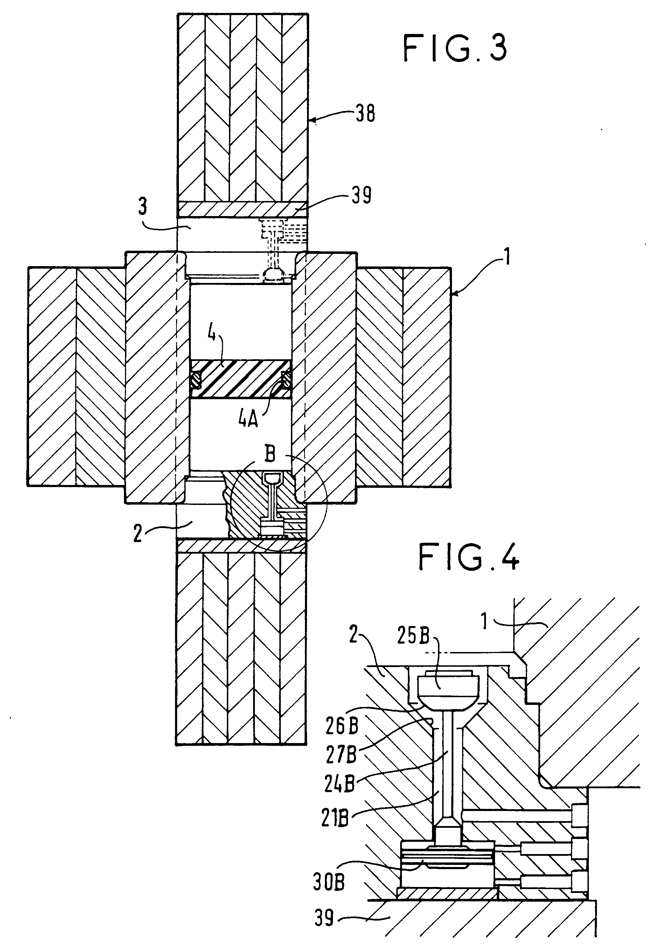

Les Fig.3 et 4 illustrent une variante de réalisation de la presse de l'invention. Les vannes 24-25 et 24A-25A disposées dans le fond supérieur 3 n'ont pas été représentées.

La figure 4 illustre dans cette variante la vanne 10 de la figure 1 et elle comprend tous les éléments des vannes précédemment décrites, cette fois affectés du suffixe B.

On distingue (Fig.4) l'alésage 21B, la tige 24B, le clapet 25B avec sa surface ovoïde 26B, le siège 27B et le vérin de commande 30B.

Les fonds 2 et 3, qui ne possèdent pas de filetage, sont montés flottants comme des bouchons. L'effort exercé sur les fonds 2 et 3 par la haute pression qui règne à l'intérieur du cylindre 1 est reporté sur une maille métallique 38 qui entoure complètement le corps 1. La poussée des fonds 2 et 3 est transmise sur la maille 38 par une pièce intercalaire 39.

Cette construction est particulièrement avantageuse car elle permet une meilleure tenue en fatigue de l'enceinte en transférant les efforts au niveau de la maille.

La solution de l'invention permet de supprimer toute tuyauterie travaillant à haute pression pour le produit à traiter à l'extérieur de la presse, ce qui accroît la sûreté du fonctionnement.

Les vannes autoclaves sont manoeuvrées au moyen d'organes de faible puissance, dans l'exemple décrit par des vérins de très petit calibre, mais en variante par tout autre organe, manuel ou motorisé.

La durée d'un cycle de traitement du produit est raccourcie de manière notable puisque la section de passage des vannes 10, 12 et 14 (figure 1) peut être très importante puisqu'elles sont incorporées au sein de pièces, les fonds 2 et 3, calculées de toute façon pour la haute pression.

L'efficacité du lavage effectué par un fluide de lavage qui emprunte le même circuit que le produit à traiter est beaucoup plus efficace car il peut être effectué à grand débit donc avec de fortes vitesses locales.

## Revendications

1. Presse isostatique, notamment pour le traitement sous haute pression de produits alimentaires pharmaceutiques ou cosmétiques, comprenant un cylindre (1) fermé hermétiquement par un fond inférieur (2) et un fond supérieur (3) et contenant en son intérieur un piston libre (4) de révolution partageant l'intérieur du cylindre en une première chambre (5) et en une seconde chambre (6), ladite première chambre (5) étant munie d'une conduite (7) d'alimentation en fluide de pressurisation équipée d'une première vanne (8) et de moyens de vidange équipés d'une seconde vanne (10) et la seconde chambre(6) étant munie de moyens d'alimentation et de vidange pour le produit à traiter équipés respectivement d'une troisième vanne et d'une quatrième vanne, caractérisée en ce que lesdites seconde, troisième et quatrième vannes comportent chacune un siège de vanne (27, 27A, 27B) situé à l'extrémité interne d'un alésage (21, 21A, 21B) d'alimentation ou de vidange de la chambre correspondante (5, 6) traversant le fond correspondant supérieur (3) ou inférieur (2), ledit siège coopérant avec un clapet (24-25, 24A-25A, 24B, 25B) disposé intérieurement audit cylindre (1).

2. Presse selon la revendication 1, caractérisé en ce que l'alésage (21B, 21, 21A) est pratiqué à travers le fond (2, 3) qui est monté flottant sur le cylindre et coopère avec une maille (38) pour le report des efforts exercés par le fluide sous pression dans le cylindre.

3. Presse selon la revendication 1, caractérisé en ce que l'étanchéité entre le fond (2, 3) et le cylindre (1) est assurée par un systéme de joints (32, 33) disposé dans un plan (37) sensiblement confondu avec le plan (36) où s'opère l'étanchéité entre le siège (27, 27A) et le clapet (25, 25A).

## Patentansprüche

1. Isostatische Presse, insbesondere für die Bearbeitung bei Hochdruck von Nahrungsmittelprodukten, Pharmazeutika oder Kosmetika, umfassend einen Zylinder (1), der hermetisch durch einen unteren Boden (2) und einen oberen Boden(3) verschlossen ist und in seinem Inneren einen freien drehbaren Kolben (4) enthält, der das Innere des Zylinders in eine erste Kammer (5) und eine zweite Kammer (6) unterteilt, wobei die erste Kammer (5) mit einer Leitung (7) zur Versorgung mit Druckfluid ausgestattet ist, welche ein erstes Ventil (8) aufweist, und Einrichtungen zur Entleerung, ausgestattet mit einem zweiten Ventil (10) und einer zweite Kammer (6), die mit Einrichtungen zur Befüllung und Entleerung für das zu bearbeitenden Produkts versehen ist, jeweils ausgestattet mit einem dritten Ventil und einem vierten Ventil, **dadurch gekennzeichnet, dass** die zweiten, dritten und vierten Ventile jeweils einen Ventilsitz (27, 27A, 27B) umfassen, der am inneren Ende einer Bohrung (21, 21A, 21B) zur Befüllung oder Entleerung der korrespondierenden Kammer (5, 6) angeordnet ist, welche den korrespondierenden oberen (3) oder unteren (2) Boden durchdringt, wobei der Sitz mit einem Stopfen (24-25, 24A-25A, 24B-25B) zusammenwirkt, der im Inneren des Zylinders (1) angeordnet ist.

2. Presse nach Anspruch 1, **dadurch gekennzeichnet, dass** die Bohrung (21B, 21, 21A) in dem Boden (2, 3) ausgebildet ist, der schwimmend in dem Zylinder montiert ist und mit einer Spange (38) für die Übertragung der ausgeübten Kräfte durch das unter Druck stehende Fluid in dem Zylinder zusammenwirkt.

3. Presse nach Anspruch 1, **dadurch gekennzeichnet, dass** die Dichtheit zwischen dem Boden (2, 3) und dem Zylinder (1) durch ein System von Dichtungen (32, 33) sichergestellt ist, die in einer Ebene (37) angeordnet sind, welche im Wesentlichen mit der Ebene (36) zusammenfällt, auf der die Dichtung zwiechen dem Sitz (27 27A) und dem Stopfen (25, 25A) erfolgt.

## Claims

1. An isostatic press, in particular for highpressure treatment of food, pharmaceutical or cosmetic products, the press including a cylinder (1) closed and hermetically sealed by a bottom end wall (2) and a top end wall (3) and containing inside it a circular free piston (4) dividing the interior of the cylinder into a first chamber (5) and a second chamber (6), said first chamber (5) being provided with a pressurising fluid feed pipe (7) equipped with a first valve unit (8) and draining means equipped with a second valve unit (10) and the second chamber (6) being provided with feed and drain means for the product to be treated respectively equipped with a third valve unit and a fourth valve unit, characterised in that said second, third and fourth valve units each include a seat (27, 27A, 27B) situated at the inside end of a feed or drain bore (21, 21A, 21B) of the corresponding chamber (5, 6) passing through the corresponding top end wall (3) or bottom end wall (2), said seat cooperating with a valve (24-25, 24A-25A, 24B, 25B) disposed inside said cylinder (1).

2. A press according to claim 1, characterised in that the bore (21B, 21, 21A) is formed through the end wall (2, 3) which is mounted floating on the cylinder and cooperates with a ring (38) to transfer forces exerted by the fluid under pressure in the cylinder.

3. A press according to claim 1, characterised in that the seal between the end wall (2, 3) and the cylinder (1) is provided by a system of seals (32, 33) disposed in a plane (37) substantially coincident with the plane (36) in which the seal is effected between the seat (27, 27A) and the valve (25, 25A).
